# NEUE EUROPÄISCHE PATENTSCHRIFT

(11) **EP 0 550 384 B2**
(45) Veröffentlichungstag und Bekanntmachung des Hinweises auf die Entscheidung über den Einspruch: **02.02.2005**
(45) Hinweis auf die Patenterteilung: 01.10.1997
(21) Anmeldenummer: 92810953.7
(22) Anmeldetag: 04.12.1992
(51) Int. Cl.: A61F 9/06, G02F 1/133, G02C 7/10

(54) **Blendschutzvorrichtung**
Ante-glare device
Dispositif anti-éblouissement

(30) Priorität: 31.12.1991 CH 386691
(43) Veröffentlichungstag der Anmeldung: 07.07.1993
(73) Patentinhaber: Sellstrom Manufacturing Co., Palatine, IL 60067 (US)
(72) Erfinder: Gunz, Stefan, CH-8820 Wädenswil (CH); Ghisleni, Livio, CH-8832 Wilen (CH)
(74) Vertreter: Scheuzger, Beat Otto

(56) Entgegenhaltungen:
- EP-A- 0 349 665
- FR-A- 2 293 188
- FR-A- 2 373 808
- FR-A- 2 379 127
- US-A- 29 684
- US-A- 3 575 491
- US-A- 3 700 306
- US-A- 4 039 254
- US-A- 4 155 122
- US-A- 4 279 474

## Beschreibung

Die vorliegende Erfindung betrifft ein Verfahren zum Betrieb einer elektrooptischen Blendschutzvorrichtung gemäss Präambel des Anspruch 1, sowie eine zur Durchführung dieses Verfahrens geeignete Blendschutzvorrichtung.

Blendschutzvorrichtungen sind allgemein bekannt und werden bevorzugt in der Schweiss- und Schneidbrennertechnik eingesetzt. In der Regel wird bei diesen Blendschutzvorrichtungen die Strahlung oberhalb 780 nm (IR) und unterhalb 365 nm (UV) weggefiltert und nur die Strahlung im sichtbaren Bereich abgeblendet.

Bekannt und beispielsweise in der FR-2 293 188 beschrieben, sind insbesondere Blendschutzscheiben, die aus einem UV-Filter, einem IR-Filter, einem Polarisator und einem Analysator, zwischen denen ein elektrooptisches Element liegt, aufgebaut sind. Das elektrooptische Element dreht dabei die Polarisationsrichtung des vom Polarisator polarisierten Lichtes in eine Richtung für welche der Analysator undurchlässig ist. Damit kann innerhalb von einigen zehntel Sekunden eine ausreichende Verdunkelung erzielt werden. Jedoch wird der Benutzer während dieser Abblendzeit intensiv geblendet und ist damit nur ungenügend geschützt.

Es sind deshalb auch schon, wie beispielsweise in der US-3 575 491 beschrieben, elektronische Schaltungen zum Betrieb von Flüssigkristallzellen entwickelt worden, mit welchen die Umschaltzeiten der Flüssigkristallzellen in den Millisekunden-Bereich zu liegen kommen. Dazu wird an die Flüssigkristallzelle eine mit einer Frequenz von über 60 Hertz schwankende elektrische, hohe Spannung angeschlossen. Leider ist der Betrieb dieser Vorrichtungen mit einer hohen elektrischen Leistung verbunden und werden die Eigenschaften der verwendeten Flüssigkristallzellen dadurch rasch verändert.

In der US 4.279.474 wird eine Blendschutzbrille mit einer variablen Verdunkelung beschrieben, deren Flüssigkristallzellen mit einer hohen Spannung und einer Wechselfrequenz betrieben werden, die oberhalb 10 Hz und vorzugsweise oberhalb der für das Auge kritischen Flimmerwahrnehmungsgrenze von 32 Hz, liegt. Derartige Blendschutzbrillen haben sich nicht durchgesetzt, weil sich deren hoher Stromverbrauch für eine autonome Stromversorgung nicht eignet.

Diese und alle anderen bisher bekannten Blendschutzvorrichtungen zeichnen sich insbesondere durch einen hohen Stromverbrauch aus. Es braucht hier auf die Nachteile von Geräten mit instabilen oder rasch verbrauchten Spannungsquellen nicht näher eingegangen zu werden.

Da die heute gebräuchlichen Blendschutzvorrichtungen ihre meist mehreren hintereinander geschalteten Flüssigkristallzellen im steilsten Bereich der Transmissionscharakteristik dieser Flüssigkristallzellen betreiben, wirken die starke Temperaturabhängigkeit und die starke Spannungsabhängigkeit dieser Charakteristik äussert nachteilig. Insbesondere erschweren diese Abhängigkeiten in der Praxis den Einsatz von selbsttätig einschaltenden Blendschutzvorrichtungen und machen eine Kompensation dieser Transmissionsänderungen erforderlich.

Ein weiteres bis heute ungelöstes Problem besteht auch in dem von den Flüssigkristallzellen selbst erzeugten Streulicht, welches von den Polarisatoren nicht eliminiert werden kann.

Aus dem Bestreben der modernen Industrie betriebssichere, einfache und unterhaltsfreundliche Lichtschutzschilde zu schaffen, stellt sich der vorliegenden Erfindung die Aufgabe diesem Bedürfnis nachzukommen, d.h. eine Blendschutzvorrichtung zu schaffen, welche die Nachteile der bekannten Vorrichtungen nicht aufweist und insbesondere eine selbsttätig arbeitende Blendschutzscheibe so zu betreiben, dass die Blendschutzvorrichtung einen geringen Stromverbrauch aufweist, im wesenlichen temperaturunempfindlich arbeitet und wenig Eigenstreulicht erzeugt.

Diese Aufgabe wird erfindungsgemäss durch ein Verfahren gelöst, welches die Merkmale des Anspruchs 1 aufweist. Eine zur Durchführung des erfindungsgemässen Verfahrens geeignete elektronische Schaltung weist wenigstens die Merkmale des vorliegenden Anspruch 5 auf.

Beim Verfahren wird mindestens eine Flüssigkristallzelle für den Aufbau der Blendschutzscheibe verwendet und in einem Spannungsbereich betrieben bei welchem die optische Transmission dieser Flüssigkristallzeite d.h. ohne vorgeschaltetes IR und/oder UV-Filter einen Wert zwischen 0,01% und 1% aufweist. Normalerweise wird dies bei einer Spannung von 5 - 20 Volt erreicht. Handelsübliche Flüssigkristallzellen werden normalerweise in ihrem Transmissionsbereich zwischen 10% bis 90%, d.h. bei einer Spannung von 1 - 4 Volt betrieben. Erfindungsgemäss wird an diese Flüssigkristallzelle eine Wechselspannung angelegt, deren Frequenz im Bereich von 0,1 Hertz liegt. Dadurch kann gegenüber herkömmlicher Blendschutzvorrichtung der Stromverbrauch erheblich gesenkt werden.

Der Betrieb der Flüssigkristallzelle bei 0.1 Hertz bietet sich insbesondere auch deshalb an, weil schon geringste Helligkeitsänderungen im Bereich von 1 - 20 Hertz vom Auge als unangenehmes Flimmern wahrgenommen werden. Eine pulsartige Helligkeitsveränderung bei 0.1 Hertz wird jedoch nicht mehr als störend empfunden.

Durch den Betrieb der Flüssigkristallzelle im Bereich erhöhter elektrischer Spannung ist diese auf Verschiebungen der Transmissionscharakteristik, wie sie beispielsweise durch Temperaturschwankungen oder Instabilitäten der Spannungsquellen verursacht werden können, nur wenig empfindlich. Ein weiterer Vorteil aus dem erfindungsgemässen Betrieb der Flüssigkristallzelle ergibt sich aus einer nennenswerten Streulichtreduktion, wie sie jede Flüssigkristallquelle beim Anlegen hoher Spannungen zeigt. Ausserdem führt das erfindungsgemässe Betriebsverfahren dazu, dass im Gegensatz zum üblichen Betrieb im Transmissionsbereich zwischen 10% und 90%, der durch die Wechselspannung erzeugte Schaltumbruch einen kaum sichtbaren Helligkeitseinbruch erzeugt. Damit weist die vorliegende Blendschutzvorrichtung nicht nur einen erheblich geringeren Stromverbrauch auf, sondern verringert die Häufigkeit und Intensität des wechselspannungsbedingten Helligkeitseinbruchs und damit die Gefahr hinlänglich bekannter Augenirritationen resp. -verletzungen.

Es sei an dieser Stelle auch darauf hingewiesen, dass moderne Schweiss- oder Schneidgeräte pulsartige Lichtemissionen, vorzugsweise im Bereich zwischen 1 - 200 Hertz erzeugen. Mit dem erfindungsgemässen Verfahren lassen sich somit auch unerwünschte Interferenzen zwischen der Betriebsfrequenz der Blendschutzvorrichtung und der Arbeitsfrequenz obiger Geräte ausschalten.

In einer bevorzugten Ausführungsform dieser Schaltung werden für die Energieversorgung Solarzellen verwendet. Damit wird die Servicefreundlichkeit der erfindungsgemässen Blendschutzvorrichtung weiter erhöht.

In einer Weiterbildung der erfindungsgemässen Schaltung ist ein Speicher, insbesondere mit einer Flip-Flop-Schaltung, vorgesehen, welcher auch bei kurzzeitigem Betrieb der Blendschutzvorrichtung eine atternierend gepolte Startspannung gewährleistet.

Im folgenden soll die Erfindung an einem Beispiel und mit Hilfe der Figuren näher erläutert werden.

Es zeigen:
- Figur 1: eine Schutzmaske mit einer Blendschutzvorrichtung,
- Figur 2: eine Transmissioncharakteristik einer handelsüblichen Flüssigkristallzelle,
- Figur 3: ein Blockschema für eine erfindungsgemässe elektronische Schaltung.

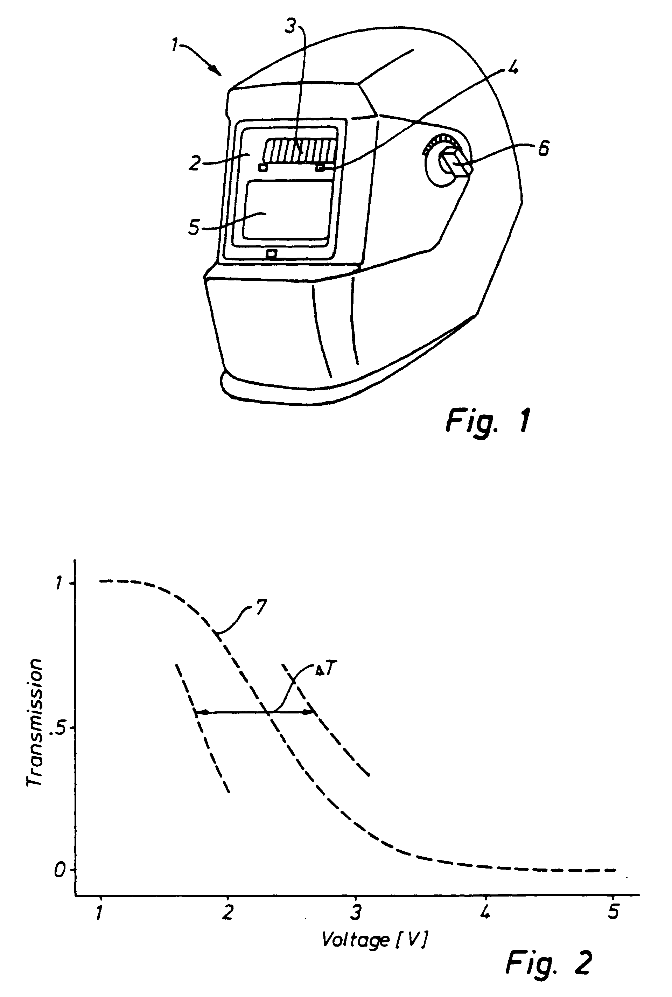

Figur 1 zeigt eine Blendschutzmaske 1 mit einer Blendschutzvorrichtung 2, wie sie heute bereits verwendet wird. Diese Blendschutzvorrichtung 2 ist als Kassette ausgebildet und kann rasch und zuverlässig ausgewechselt werden. Die der abzublendenden Lichtquelle zugewandte Seite dieser Kassette 2 weist eine Serie von Solarzellen 3 auf, mindestens einen Fotosensor 4 und eine Blendschutzscheibe 5. Diese Blendschutzscheibe 5 ist in bekannter Weise aus mehreren Elementen sandwichartig aufgebaut und besteht in einer einfacheren Ausführungsform aus einem mit einer Kratzschutzschicht versehenen ersten Polarisator, der gleichzeitig auch als UV-Filter wirkt, einem IR-Filter, mindestens einer Flüssigkristallzelle, einem zweiten ebenfalls mit einer Kratzschutzschicht versehenen Polarisator, sowie allenfalls einer Lupe. Bei dieser Auführungsform kann die Abdunklung mit Hilfe eines Regelknopfes 6 manuell nachgeregelt werden.

Figur 2 zeigt eine für handelsübliche TN-LCD's (Twisted Nematic Liquid Cristal Display) typische Transmissionscharakteristik 7. Diese Charakteristik macht deutlich, dass diese Flüssigkristallzellen bei niederen Spannungen vollständig lichtdurchlässig sind und im Bereich von 1 - 4 Volt undurchlässig werden. Für Spannungen oberhalb 4 Volt beträgt die Transmission bei dieser Flüssigkristallzelle weniger als 1%. Transmissionswerte von weniger als 1% sind aber gerade für die vorliegende Anwendung erforderlich. Es wird nun deutlich, dass die derartig verwendeten Flüssigkristallzellen im Betrieb unverhältnismässig viel Strom verbrauchen, insbesondere wenn damit eine hohe Extinktion, d.h. von mehr als 99%, erzielt werden soll. Üblicherweise werden deshalb mehrere Flüssigkristallzellen hintereinander geschaltet. Damit kann dieses Problem gemildert werden, ist aber so nicht zufrieden stellend gelöst. Demgegenüber kann bei der vorliegenden Erfindung mit nur einer Flüssigkristallzelle dieselbe Wirkung erzielt werden. Erfindungsgemäss wird dazu eine handelsübliche Flüssigkristallzelle mit einer ungewöhnlich niederen Wechselfrequenz, d.h. ca. 0,1 Hertz, betrieben. Dadurch kann die durch das normalerweise notwendige Umpolarisieren der Flüssigkristallzellen benötigte Betriebsleistung reduziert und die Leistungszunahme aus der relativ hohen Betriebsspannung überraschend gut kompensiert werden. Tatsächlich ist die Leistungszunahme aus der erfindungsgemässen Betriebsspannung kleiner als die aus der niederen Betriebsfrequenz resultierende Leistungseinsparung.

Durch die erhöhte Spannung wird gleichzeitig eine Reduktion des von der Flüssigkristallzelle erzeugten Streulichtes erzielt. Ausserdem ist der Helligkeftseinbruch beim Umschaltvorgang geringer, da mit der erhöhten Spannung auch die Flüssigkristallzelle rascher verdunkelt.

Für den erfindungsgemässen Betrieb geeignet gestaltete Flüssigkristallzellen und insbesondere niederfrequent betreibbare Flüssigkristallzellen können unter den erfindungsgemässen Bedingungen mehrere Jahre einwandfrei arbeiten.

Aus der Figur 2 ist auch die Verschiebung der Transmissionscharakteristik bei Temperaturänderungen ersichtlich. Dabei kann eine Änderung von ca. 20° Celsius zu Transmissionsverschiebungen von bis zu 50% führen. Beim erfindungsgemässen Betrieb der Flüssigkristallzelle ist diese Verschiebung kaum merkbar und bewirkt deshalb auch keine Änderungen in der Transmission.

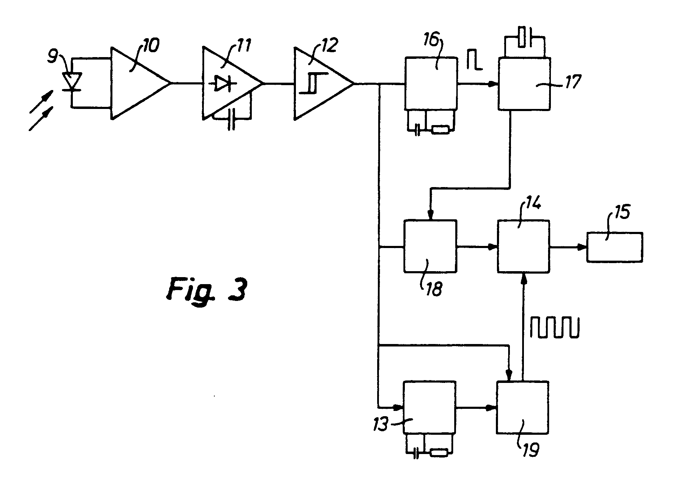

Figur 3 zeigt ein Blockschema für eine elektronische Schaltung wie sie beispielsweise zur Durchführung des erfindungsgemässen Verfahrens geeignet ist. Dabei wird das von einem Fotodetekor 9 erzeugte Signal von Schaltungen 10 und 11 verstärkt und gleichgerichtet und einem Schwellwertschalter 12 zugeführt. Das von diesem Schwellwertschalter 12 erzeugte Signal wird einem mit einem Teiler versehenen Oszillator 13 zugeführt, welcher die Wechselfrequenz für einen LCD-Treiber 14 erzeugt, an welchem eine Flüssigkristallzelle 15 angeschlossen ist. Zur Erhöhung der Schaltgeschwindigkeit wird das vom Schwellwertschalter 12 erzeugte Signal auch einem Pulsformer 13 und einem DC/DC-Spannungsverstärker 17 zugeführt, welche das Einschaltsignal des Schwellwertschalters 12 mit einer steilen Startflanke versehen. Dieses Signal wird einem Umschalter 18 zugeführt, um die Betriebsspannung, bspw. 6-12 Volt, für den Einschaltprozess sprunghaft, bspw. auf 15-30 Volt, zu erhöhen und um diese nach einer vorgegebenen Zeit, bspw. 1-20 ms, wieder auf die normale Betriebsspannung zurückzuschalten.

In einer bevorzugten Ausführungsform ist zwischen dem Oszillator 13 und dem LCD-Treiber 14 ein Speicher 19 geschältet. Dieser Speicher stellt sicher, dass die Polarität der Startspannung bei jedem Einschakvorgang geändert wird, insbesondere um elektrolytische Verschleisserscheinungen an der Flüssigkristallzelle 15 zu reduzieren.

Es versteht sich, dass die besondere Konstruktion sowohl der Blendschutzscheibe als auch der elektronischen Schaltung im Bereich des fachmännischen Könnens liegen. Insbesondere können anstelle der Fotodetektoren auch elektromagnetische oder pyroelektrische Detektoren verwendet werden, oder können die Solarzellen auch integrierender Bestandteil der Blendschutzscheibe selbst sein, oder kann die elektronische Schaltung zusätzlich manuell einstellbar sein. Insbesondere liegt die Wahl einer besonders geeigneten Flüssigkristallzelle im normalen technischen Handeln des Fachmanns.

Weiterbildungen der zur Durchführung des erfindungsgemässen Verfahrens geeigneten Mittel, insbesondere optische Beschichtungen der einzelnen Elemente der elektrooptischen Blendschutzscheibe, oder besondere Gestaltungen der Flüssigkristallzelle, insbesondere Korrosionsschutzschichten für deren Elektroden liegen ebenfalls im Bereich des fachmännischen Könnens.

Die Verwendung der erfindungsgemässen Blendschutzscheibe und deren elektronischen Schaltung in Brillen jeder Art, bzw. Modebrillen, Sonnenschutzbrillen, Diskobrillen, Autofahrer-und Pilotenbrillen ist ebenso naheliegend wie deren Anwendung in der allgemeinen LCD-Technik als Blendschutz.

## Patentansprüche

1. Verfahren zum Betrieb einer elektrooptischen Blendschutzvorrichtung für Schutzbrillen, Schutzhelme oder Schutzmasken, mit mindestens einem Detektor (9), insbesondere einer Fotodiode, mit einer mindestens eine Flüssigkristallzelle (15) umfassenden elektrooptischen Blendschutzscheibe (5), und mit einer elektronischen Schaltung zur Einstellung der optischen Transmission dieser Blendschutzscheibe (5), wobei die Flüssigkristallzelle (15) mit einer elektrischen Betriebsspannung betrieben wird, bei welcher der optische Transmissionswert dieser Flüssigkristallzelle (15) kleiner als 1% ist **dadurch gekennzeichnet, dass** die Frequenz der Betriebsspannung für den Betrieb dieser Flüssigkristallzelle (15) bei 0,1 Hertz liegt.

2. Verfahren nach Anspruch 1, **dadurch gekennzeichnet, dass** zur Einstellung der optischen Transmission der Blendschutzscheibe (5) ein vom Detektor (9) erzeugtes Signal verstärkt, gleichgerichtet und einem Schwellwertschalter (12) zugeführt wird, welcher Schwellwertschalter (12) ein Einschaltsignal generiert, welches einem Oszillator (13) zugeführt wird, welcher Oszillator (13) ein Wechselspannungssignal für einen LCD-Treiber (14), an welchem die Flüssigkristallzelle (15) angeschlossen ist, erzeugt.

3. Verfahren nach Anspruch 2, **dadurch gekennzeichnet, dass** das vom Schwellwertschalter erzeugte Signal einer Schaltung zur Beschleunigung des Einschaltvorganges zugeführt wird.

4. Verfahren nach Anspruch 2, **dadurch gekennzeichnet, dass** dem LCD-Treiber (14) eine alternierend gepolte Startspannung zugeführt wird.

5. Elektronische Schaltung geeignet zur Durchführung des Verfahrens gemäss Anspruch 1, welche Schaltung mindestens einen Detektor (9), einen Schwellwertschalter (12), einen Oszillator (13), einen Flüssigkristallzellen-Treiber (14), eine Flüssigkristallzelle (15) sowie eine angepasste Energieversorgung aufweist, welcher Treiber (14) eine Betriebsspannung erzeugt, bei welcher der optische Transmissionswert der Flüssigkristallzelle kleiner als 1% ist **dadurch gekennzeichnet, dass** der Oszillator (13) den Treiber (14) mit einer Frequenz bei 0.1 Hertz, taktet

6. Elektronische Schaltung nach Anspruch 5, **dadurch gekennzeichnet, dass** eine Schaltung zur Beschleunigung des Einschaltvorganges vorgesehen ist.

7. Elektronische Schaltung nach Anspruch 5, **dadurch gekennzeichnet, dass** deren elektronische Schaltung für die Erzeugung einer alternierend gepolten Startspannung einen, insbesondere mit einer Flip-Flop-Schaltung versehenen Speicher (19) umfasst.

## Claims

1. Method for operating an electro-optical glare protection device for protective glasses, protective helmets or protective masks, with at least one detector (9), in particular a photo diode, with an electro-optical glare protection plate (5) comprising at least one liquid crystal cell (15), and with an electronic circuit for controlling the optical transmission of said glare protection plate (5), whereby the liquid crystal cell (15) is operated by means of an electrical operating voltage at which voltage the optical transmission value of said liquid crystal cell (15) is less than 1%, **characterized in that** the frequency of the operating voltage for the operation of said liquid crystal cell (15) lies at around 0.1 Hertz.

2. Method according to claim 1, **characterized in that** for controlling the optical transmission of the glare protection plate (5), a signal produced by the detector (9) is amplified, rectified and is fed to a threshold value switch (12), said threshold value switch (12) generating a switch-on signal which is fed to an oscillator (13), said oscillator (13) generating an alternating voltage signal for an LCD-driver (14) to which the liquid crystal cell (15) is connected.

3. Method according to claim 2, **characterized in that** the signal generated by the threshold value switch is fed to a circuit for accelerating the switch-on procedure.

4. Method according to claim 2, **characterized in that** an alternatingly polarized starter voltage is fed to the LCD-driver (14).

5. Electronic circuit suitable for carrying out the method according to claim 1, said circuit comprising at least one detector (9), a threshold value switch (12), an oscillator (13), a liquid crystal cell driver (14), a liquid crystal cell (15), as well as an adapted energy supply, said driver (14) producing an operating voltage at which voltage the optical transmission value of the liquid crystal cell is less than 1%, **characterized in that** the oscillator (13) phases the driver (14) at a frequency of around 0.1 Hertz.

6. Electronic circuit according to claim 5, **characterized in that** a switch for accelerating the switch-on procedure is foreseen.

7. Electronic circuit according to claim 5, **characterized in that** its electronic circuit for generating an alternatingly polarized starter voltage comprises a memory (19), in particular a memory with a flip-flop-switch.

## Revendications

1. Procédé pour exploiter un dispositif anti-éblouissement électro-optique pour des lunettes de protection, des casques de protection ou des masques de protection, avec au moins un détecteur (9), notamment une photodiode, avec une plaque anti-éblouissante (5) électro-optique comprenant au moins une cellule à cristaux liquides (15) et avec un circuit électronique pour le réglage de la transmission optique de cette plaque anti-éblouissante (5), la cellule à cristaux liquides (15) étant commandée par une tension de service électrique à laquelle la valeur de transmission optique de cette cellule à cristaux liquides (15) est inférieure à 1%, **caractérisé en ce que** la fréquence de la tension de service pour la commande de cette cellule à cristaux liquides (15) se situe autour de 0.1 Hertz.

2. Procédé selon la revendication 1, **caractérisé en ce que**, pour le réglage de la transmission optique de la plaque anti-éblouissante (5), un signal produit par le détecteur (9) est amplifié, redressé et amené à un commutateur à seuil (12), lequel commutateur à seuil (12) génère un signal d'enclenchement qui est amené à un oscillateur (13), lequel oscillateur (13) produit un signal de tension alternative pour un circuit d'attaque pour affichage par cristaux liquides (14) auquel est reliée la cellule à cristaux liquides (15).

3. Procédé selon la revendication 2, **caractérisé en ce que** le signal produit par le commutateur à seuil est amené à un circuit pour accélérer le processus d'enclenchement.

4. Procédé selon la revendication 2, **caractérisé en ce qu'**une tension de démarrage polarisée en alternance est amenée au circuit d'attaque pour affichage par cristaux liquides (14).

5. Circuit électronique approprié pour exécuter le procédé selon la revendication 1, lequel circuit comprend au moins un détecteur (9), un commutateur à seuil (12), un oscillateur (13), un circuit d'attaque (14) pour cellules à cristaux liquides, une cellule à cristaux liquides (15) ainsi qu'une alimentation en énergie adaptée, lequel circuit d'attaque (14) produit une tension de service avec laquelle la valeur de transmission optique de la cellule à cristaux liquides est inférieure à 1%, **caractérisé en ce que** l'oscillateur (13) fait osciller le circuit d'attaque (14) à une fréquence située autour de 0,1 Hertz.

6. Circuit électronique selon la revendication 5, **caractérisé en ce que** l'on prévoit un circuit pour accélérer le processus d'enclenchement.

7. Circuit électronique selon la revendication 5, **caractérisé en ce que** son circuit électronique pour la production d'une tension de démarrage polarisée en alternance comprend une mémoire (19), notamment une mémoire pourvue d'un montage en bascule.
